# EUROPÄISCHE PATENTANMELDUNG

(11) **EP 0 816 379 A2**
(43) Veröffentlichungstag der Anmeldung: **07.01.1998**
(21) Anmeldenummer: 97109741.5
(22) Anmeldetag: 16.06.1997
(51) Int. Cl.: C07K 14/00, A61K 38/00

(54) **Nukleinsäure-bindende Oligomere**

(30) Priorität: 27.06.1996 DE 19625782; 30.08.1996 DE 19635064
(71) Anmelder: BAYER AG, 51368 Leverkusen (DE)
(72) Erfinder: Jordan, Stephan, Dr., 51061 Köln (DE); Kosch, Winfried, Dr., 65203 Wiesbaden (DE); Kretschmer, Axel, Dr., 51429 Bergisch Gladbach (DE); Schwemler, Christoph, Dr., 42799 Leichlingen (DE); Stropp, Udo, Dr., 42781 Haan (DE)

(57) **Zusammenfassung**

Die Erfindung betrifft Verbindungen der allgemeinen Formel (I) 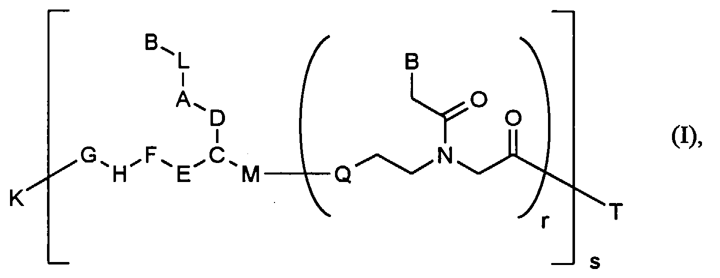 in der die einzelnen Gruppen die in der Beschreibung angegebenen Bedeutungen haben und deren Verwendung als Arzneimittel.

## Beschreibung

Peptidnukleinsäuren sind polyamidische Verbindungen, die in der Seitenkette Purine oder Pyrimidine tragen. Es handelt sich somit formal um DNA-analoge Verbindungen, bei denen das Desoxyribosephosphatgerüst durch ein Peptidrückgrat ersetzt wurde.

Das gezielte Abschalten von Genexpression durch komplementäre Nukleinsäuren, sogenannte Antisense-Oligonukleotide, stellt einen neuen Therapieansatz dar. Mögliche Anwendungen reichen von der Behandlung viraler Infektionen bis zur Therapie von Krebs (S. Agrawal, Tibtech 10, 152 (1992); W. James, Antiviral Chemistry & Chemotherapie 2, 191 (1991); B. Calabretta, Cancer Research 51, 4504 (1991)). Die Kontrolle der Genexpression erfolgt auf der Ebene von DNA und RNA und gelingt bereits mit unmodifizierten Oligonukleotiden (C. Helene, Anti-Cancer Drug Design 6, 569 (1991); E. Uhlmann, A. Peymann, Chemical Reviews 90, 543 (1990)). Diese sind jedoch aufgrund mangelnder enzymatischer Stabilität und zu geringer Aufnahme in zelluläre Systeme für therapeutische Anwendungen nicht geeignet. Therapeutische Anwendungen erfordern chemisch modifizierte Antisense-Oligonukleotide.

Neben der Antisense-Strategie kann eine Inhibition der Genexpression auch durch die Sense-Strategie erzielt werden. Dabei konkurrieren die Sense-Oligonukleotide spezifisch mit DNA-Bindungsproteinen wie z.B. Transkriptionsfaktoren (M. Blumengeld, Nucleic Acids Research 21, 3405 (1993)).

Oligonukleotide mit modifiziertem Internukleotidphosphat oder einer phosphatfreien Internukleotidverknüpfung wurden in vielen Arbeiten systematisch untersucht; ihre Synthese erwies sich jedoch als sehr aufwendig und beobachtete therapeutische Effekte als nicht ausreichend (E. Uhlmann, A. Peyman, Chemical Reviews 90, 543 (1990)).

Eine Alternative zur Modifikation oder Substitution der Phosphatgruppe in Nukleinsäuren ist der komplette Austausch von Ribose und Phosphat durch andere Rückgrate. Dieses Konzept wurde erstmals von Pitha et al. realisiert, der Ribosephosphat durch Poly-N-Vinyl-derivate ersetzte, was zu sogenannter "Plastik-DNA" führt (J. Pitha, P.O.P. Ts'O, J. Org. Chem. 33, 1341 (1968); J. Pitha, J. Adv. Polym. Sci. 50, 1 (1983)). Es erlaubt jedoch nicht den gezielten Aufbau definierter Sequenzen.

Die Synthese definierter Sequenzen gelingt, wenn anstelle von Zuckerphosphat beispielsweise ein Polyamid-Rückgrat verwendet wird, das in Analogie zur konventionellen Peptidsynthese (M. Bodanszky, Principles of Peptide Synthesis, Springer, Berlin 1984) schrittweise aufgebaut wird. Dieses Konzept wurde von verschiedenen Arbeitsgruppen unterschiedlich realisiert (J.E. Summerton et al. WO 86/05518; R. S. Varma et al. WO 92/18518; O. Buchardt et al. WO 92/20702; H. Wang, D. D. Weller, Tetrahedron Letters 32, 7385 (1991); P. Garner, J. U. Yoo, Tetrahedron Letters 34, 1275 (1993); S.-B. Huang, J. S. Nelson, D. D. Weller, J. Org. Chem. 56, 6007 (1991); EP 646 595 A1, EP 646 596 A1 u. EP 700 928 A1; EP 672 661 A1, EP 672 677 A2 und EP 672 700 A1).

Polyamid-Nukleinsäuren eignen sich ebenfalls für diagnostische und molekularbiologische Anwendungen (Buchardt et al. WO 92/20703 und Glaxo Inc. WO 93/12129).

Bei der Bearbeitung deartiger Strukturen gelang die Synthese neuer C-und N-verzweigter oligomerer Nukleinsäuren. Für diese wurde eine überraschend gute Bindung an DNA und RNA gefunden. Die Substanzen eignen sich zur Kontrolle der Genexpression und zeigen antivirale Eigenschaften. Weiterhin lassen sich derartige Substanzen in Diagnostik und Molekularbiologie zur Isolierung, Identifizierung und Quantifizierung von Nukleinsäuren verwenden.

Die Erfindung betrifft Verbindungen der allgemeinen Formel (I) 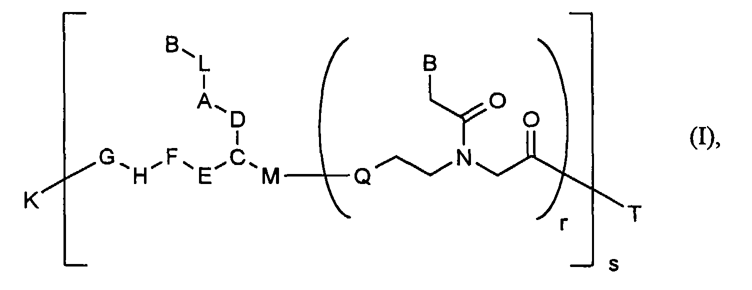 in der
- A: für -CO-, -CHR oder -CRR'- steht,
- B: für alle natürlichen oder unnatürlichen Nukleobasen, wie z.B. Thymin, Uracil, Cytosin, Adenin, Guanin, Hypoxanthin oder durch chemische Modifikation von diesen abgeleitete Derivate oder halogenierte Vorstufen von diesen, gegebenenfalls an den Aminogruppen durch Schutzgruppen wie Acetyl, Trifluoracetyl, Trichloracetyl, Benzoyl, Phenylacetyl, Benzyloxycarbonyl (Z), tert.-Butyloxycarbonyl (Boc), Allyloxycarbonyl, (9-Fluorenyl)-methoxycarbonyl (Fmoc) oder andere in der Peptid- und Nukleinsäurechemie übliche Schutzgruppen substituiert, steht,
- C: für -CH- oder -CR- steht, wobei C einheitlich S oder R konfiguriert sein kann,
- D: für -NH-, -NR-, -CH₂-, -CHR- oder -CRR'- steht,
- E: für -NH-, -NR-, -CH₂-, -CHR, -CRR'-, -O- oder -S- steht,
- A und E: miteinander über eine Alkylkette [(-CH₂)ₙ-, mit n = 0, 1, 2] verbunden sein können,
- F: für -CH₂-, -CO-, SO₂-, -SO- oder -CS- steht,
- G: für -NH-, -NR-, -O- oder -S- steht,
- H: für eine Bindung, -CH₂-CH₂- oder -CR¹R²- steht, worin
- R¹: für Wasserstoff oder Methyl steht und
- R²: für Wasserstoff, gegebenenfalls durch Amino oder Hydroxy substituiertes C₁-C₄-Alkyl oder für Mercaptomethyl, Methylthioethyl, Carboxymethyl, Carboxyethyl, Carbamoylmethyl, Carbamoylethyl, Guanidinopropyl oder für gegebenenfalls durch Amino, Nitro, Halogen, Hydroxy oder Methoxy substituiertes Phenyl oder Benzyl oder für Naphthylmethyl, Indolylmethyl, Imidazolylmethyl, Triazolylmethyl oder Pyridylmethyl steht, wobei funktionelle Gruppen gegebenenfalls geschützt vorliegen und die Gruppe -CR¹R²-stereochemisch einheitlich in der L-Form oder D-Form konfiguriert sein kann,
- G und H: miteinander über eine Alkylkette [(-CH₂)ₙ- mit n = 3 oder 4] verbunden sein können,
- L: für (-CH₂)ₚ- mit p = 0, 1 oder 2, -CHR- oder -CRR'- steht,
- M: für -CH₂-, -CO-, -SO₂-,-SO- oder -CS- steht,
- Q: für NH, O, S oder NR³ steht, worin
- R³: für C₁-C₈-Alkyl, C₂-C₈-Alkenyl, Phenyl, Pyridyl, Benzyl oder Naphthylmethyl steht,
- K: für Wasserstoff, Carrier-System, löslichkeitsvermittelnde Gruppe oder Acylrest einer natürlichen oder unnatürlichen Aminosäure (C-verknüpfte Aminosäure) steht,
- T: für Hydroxy, Carrier-System, löslichkeitsvermittelnde Gruppe oder amidisch (N-) verknüpfte natürliche oder unnatürliche Aminosäure steht,
- R und R': jeweils unabhängig voneinander für OH, Methyl, Ethyl, n-Propyl, iso-Propyl, n-Butyl, iso-Butyl, sec.-Butyl oder tert.-Butyl, Aralkyl wie beispielsweise Benzyl, α-Naphthylmethyl oder β-Naphthylmethyl oder gegebenenfalls durch Methyl, Halogen oder NO₂ substituiertes Aryl oder Hetaryl wie beispielsweise Phenyl, 2-Pyridyl oder 4-Pyridyl steht,
- r: für 0 oder 1 steht und
- s: für einen Wert von 1 bis 30 steht.

Für den Fall, daß in allen Monomeren r = 1 ist, kommt dieser Baustein alternierend vor und hat einen Anteil von 50 % am Gesamtmolekül. Wenn r in einzelnen Monomeren 0 (null) ist, reduziert sich der Anteil dieses Bausteins entsprechend auf beispielsweise 40, 30 oder 20 %. Im Gesamtmolekül soll dieser Baustein mindestens einmal vorkommen.

Die erfindungsgemäßen Verbindungen sind durch die Formel (I) allgemein definiert.
- A: steht bevorzugt für -CO-, -CHR- oder -CRR'-.
- B: steht bevorzugt für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert sind.
- C: steht bevorzugt für -CH- oder -CR-, wobei C einheitlich S oder R konfiguriert sein kann.
- D: steht bevorzugt für -NH-, -NR-, -CH₂-, -CHR- oder -CRR'-.
- E: steht bevorzugt für -NH-, -NR-, -CH₂-, -CHR-, -CRR'- oder -O-.
- A und E: können bevorzugt miteinander über eine Alkylkette [(-CH₂)ₙ-, mit n = 0, 1, 2] verbunden sein ,
- F: steht bevorzugt für -CH₂-, -CO- oder -CS-.
- G: steht bevorzugt für -NH-, -NR- oder -O-.
- H: steht bevorzugt für eine Bindung, -CH₂-CH₂- oder -CR¹R²-, worin
- R¹: für Wasserstoff oder Methyl steht und
- R²: für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl, 4-Methoxybenzyl, 2-Nitrobenzyl, 3-Nitrobenzyl, 4-Nitrobenzyl, 2-Aminobenzyl, 3-Aminobenzyl, 4-Aminobenzyl, 3,4-Dichlorbenzyl, 4-Iodbenzyl, α-Naphthylmethyl, β-Naphthylmethyl, 3-Indolylmethyl, 4-Imidazolylmethyl, 1,2,3-Triazol-1-yl-methyl, 1,2,4-Triazol-1-yl-methyl, 2-Pyridylmethyl oder 4-Pyridylmethyl steht, wobei funktionelle Gruppen gegebenenfalls geschützt vorliegen und die Gruppe -CR¹R²- stereochemisch einheitlich in der L-Form oder D-Form konfiguriert ist.
- G und H: können bevorzugt miteinander über eine Alkylkette [(-CH₂)ₙ- mit n = 3 oder 4] verbunden sein.
- L: steht bevorzugt für (-CH₂)ₚ- mit p = 0, 1 oder 2, -CHR- oder -CRR'-.
- M: steht bevorzugt für -CH₂-, -CO-, oder -CS-.
- Q: steht bevorzugt für NH, O oder NR³ steht, worin
- R³: für C₁-C₄-Alkyl, C₂-C₄-Alkenyl, Phenyl, 2-Pyridyl, 4-Pyridyl, Benzyl, α-Naphthylmethyl oder β-Naphthylmethyl steht.
- K: steht bevorzugt für Wasserstoff, Carrier-System, löslichkeitsvermittelnde Gruppe oder Acylrest einer natürlichen oder unnatürlichen Aminosäure (C-verknüpfte Aminosäure).
- T: steht bevorzugt für Hydroxy, Carrier-System, löslichkeitsvermittelnde Gruppe oder amidisch (N-) verknüpfte natürliche oder unnatürliche Aminosäure.
- R und R': stehen bevorzugt jeweils unabhängig voneinander für OH, Methyl, Ethyl, n-Propyl oder n-Butyl, Benzyl oder gegebenenfalls durch Methyl, Fluor, Chlor, Brom oder NO₂ substituiertes Phenyl.
- r: steht bevorzugt für 0 oder 1.
- s: steht bevorzugt für einen Wert von 1 bis 20.
- A: steht besonders bevorzugt für -CO-, -CHR- oder -CRR'-.
- B: steht besonders bevorzugt für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert sind.
- C: steht besonders bevorzugt für -CH-, -CR-, wobei C einheitlich S oder R konfiguriert sein kann.
- D: steht besonders bevorzugt für -NH-, -N(CH₃)-, -CH₂-, -CHR- oder - CRR'-.
- E: steht besonders bevorzugt für -NH-, -NR-, -CH₂- oder -CHR-.
- F: steht besonders bevorzugt für -CH₂-, -CO- oder -CS-.
- G: steht besonders bevorzugt für -NH- oder -N(CH₃)-.
- H: steht besonders bevorzugt für eine Bindung, -CH₂-CH₂- oder -CR¹R²-, worin
- R¹: für Wasserstoff oder Methyl steht und
- R²: für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl steht, wobei funktionelle Gruppen gegebenenfalls geschützt vorliegen und die Gruppe -CR¹R²- stereochemisch einheitlich in der L-Form oder D-Form konfiguriert ist.
- L: steht besonders bevorzugt für (-CH₂)ₚ- mit p = 0, 1 oder 2, -CHR-oder -CRR'-.
- M: steht besonders bevorzugt für -CH₂-, -CO-, oder -CS-.
- Q: steht besonders bevorzugt für NH, O oder NR³ steht, worin
- R³: für Methyl, Ethyl, n-Propyl, iso-Propyl, n-Butyl, iso-Butyl, sec.-Butyl, tert.-Butyl, Vinyl, Allyl, Phenyl, 2-Pyridyl, 4-Pyridyl, Benzyl, α-Naphthylmethyl oder β-Naphthylmethyl steht.
- K: steht besonders bevorzugt für Wasserstoff, Carrier-System, löslichkeitsvermittelnde Gruppe oder Acylrest einer natürlichen oder unnatürlichen Aminosäure (C-verknüpfte Aminosäure).
- T: steht besonders bevorzugt für Hydroxy, Carrier-System, löslichkeitsvermittelnde Gruppe oder amidisch (N-) verknüpfte natürliche oder unnatürliche Aminosäure.
- R und R': steht besonders bevorzugt jeweils unabhängig voneinander für OH, Methyl oder Benzyl.
- r: steht besonders bevorzugt für 0 oder 1.
- s: steht besonders bevorzugt für einen Wert von 2 bis 15.

Die aus der Peptidchemie bekannten Schutzgruppen sind z.B. aufgeführt in T. W. Greene, P. G. M. Wuts, Protective Groups in Organic Synthesis, 2^{nd} Ed., John Wiley & Sons, New York 1991.

Carrier-System bedeutet ein zellspezifisches Bindungs- und Erkennungs-Agens, das spezifisch an die Zelloberfläche bindet und das die Internalisierung der der Erfindung zugrunde liegenden Nukleinsäure-bindenden Oligomeren bewirkt. Die Internalisierung kann auf verschiedenem Wege, z.B. durch Endocytose oder aktive Transportmechanismen erfolgen.

Die Struktur der Zelloberfläche kann ein Protein, Polypeptid, Kohlenhydrat, Lipid oder eine Kombination daraus sein. Typischerweise wird die Zellaufnahme durch Oberflächenrezeptoren bewirkt. Deshalb kann das Bindungs- und Erkennungs-Agens ein natürlicher oder synthetischer Ligand eines Rezeptors sein.

Der Ligand kann ein Protein, Polypetid, Kohlenhydrat, Lipid oder eine Kombination von diesen sein, ausgestattet mit funktionellen Gruppen, die so angeordnet sind, daß sie durch die Zelloberflächen-Struktur erkannt werden können. Es kann sich auch um eine Komponente oder die Gesamtheit eines biologischen Organismus handeln, z.B. eines Virus, einer Zelle oder um artifizielle Transportsysteme, wie z.B. Liposomen. Es kann sich weiterhin um einen Antikörper oder ein Analogon eines Antikörpers handeln.

Für die Adressierung an unterschiedliche Zellen müssen verschiedene Liganden eingesetzt werden.

Als Liganden für die Adressierung an Makrophagen kommen bevorzugt Kohlenhydrate, wie z.B. Mannose, Polykationen, wie z.B. Polylysine, Polyarginine, Polyornithine, basische Proteine, wie z.B. Avidin, sowie Glycopeptide, Peptide oder Lipopeptide in Frage (G.Y. Chu et al., WO 9304701).

Mit löslichkeitsvermittelnden Gruppen sind funktionelle Gruppen gemeint, die die Löslichkeit in Wasser vermitteln. Dabei kann es sich z.B. um Ester oder Amide von Aminosäuren, Hydroxycarbonsäuren, Aminosulfonsäuren, Hydroxysulfonsäuren oder Diaminen handeln. Bevorzugt sind Amide von Diaminocarbonsäuren, wie Ornithin, Lysin oder 2,4-Diaminobuttersäure.

Wenn ein Carrier-System oder eine löslichkeitsvermittelnde Gruppe für den Rest K stehen, ist dessen Anknüpfungspunkt beispielsweise, analog zur N-terminalen Verknüpfung einer Endgruppe in der Peptidchemie, eine Acylgruppe (d.h. einschließlich Rest G besteht die Verknüpfung über eine Amid, Ester oder Thioesterfunktion) oder im Falle von Kohlehydratresten bevorzugt der Sauerstoff einer glykosidischen Hydroxygruppe. Für Rest T stehend, ist die Verknüpfungsstelle analog zu einer am C-terminalen Ende in der Peptidchemie verbundenen Endgruppe ein Sauerstoff- oder Stickstoffatom einer Amino- oder Hydroxygruppe des Carrier-Systems oder der löslichkeitsvermittelnden Gruppe.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Beispiele für die erfindungsgemäßen Verbindungen der Formel (I) sind Peptid-Nukleinsäuren der Formel (I-a) mit variablem Anteil an α-methylierten α,ω-Diaminocarbonsäure-Bausteinen. 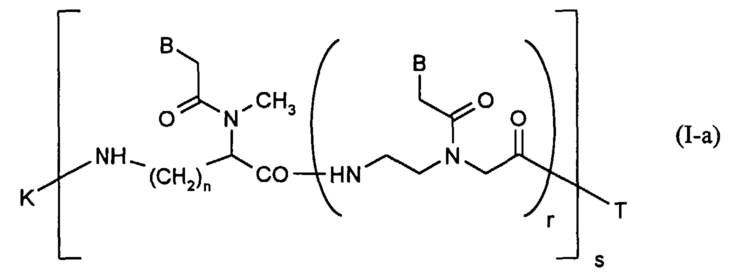
- n =: 2, 3, 4
- r =: 0, 1; mindestens einmal 1
- s =: 1 - 30
- B =: 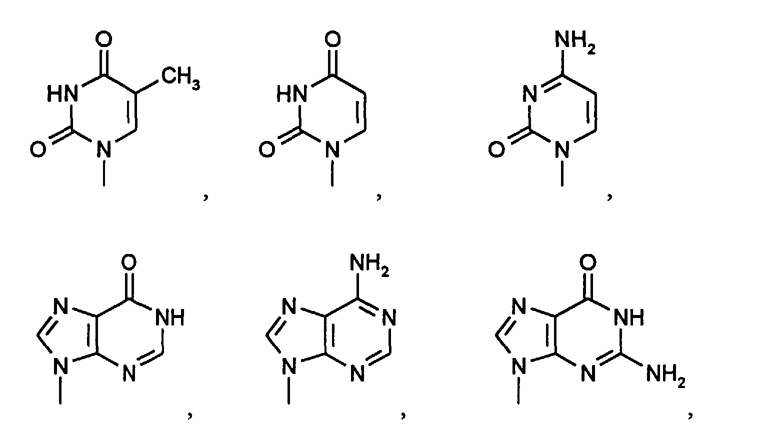 usw.

Bei Verbindungen dieses Typs ist das Zuckerphosphat-Rückgrat der natürlichen Nukleinsäuren durch α-methylierte α,ω-Diaminocarbonsäuren wie *N*-Methyllysin, *N*-Methylomithin oder *N*-Methyl-2,4-diaminobuttersäure verknüpft mit Aminoethylglycinbausteinen ersetzt. Die Nukleobasen sind über Acylspacer an die α-Aminogruppen gebunden.

### Beschreibung der Experimente

### Allgemeiner Teil

### Synthesen von monomeren Bausteinen

Ausgehend von käuflichen δ-*N*-Boc-geschütztem L-Ornithinmethylester **1** wird über reduktive Aminierung (z.B. Benzaldehyd in Gegenwart von Natriumcyanoborhydrid) eine Benzylschutzgruppe an der α-Aminogruppe eingeführt (Verb. **2**). Anschließend wird α-*N*-methyliert (z.B. durch Umsetzung mit Methyliodid in Gegenwart von Kaliumcarbonat zu **3**) und danach die *N*-Benzylschutzgruppe wieder abhydriert. Die Einführung der Nukleobasen gelingt beispielsweise durch Reaktion der Verbindung **4** mit 1-Carboxymethylthymin in Gegenwart von *N*'-(3-Dimethylaminopropyl)-*N*-ethylcarbodiimid (EDCI) und 1-Hydroxy-1H-benzotriazol (HOBt). Der zur Oligomerisierung geeignete Baustein **6** wird durch Verseifung von **5** erhalten.

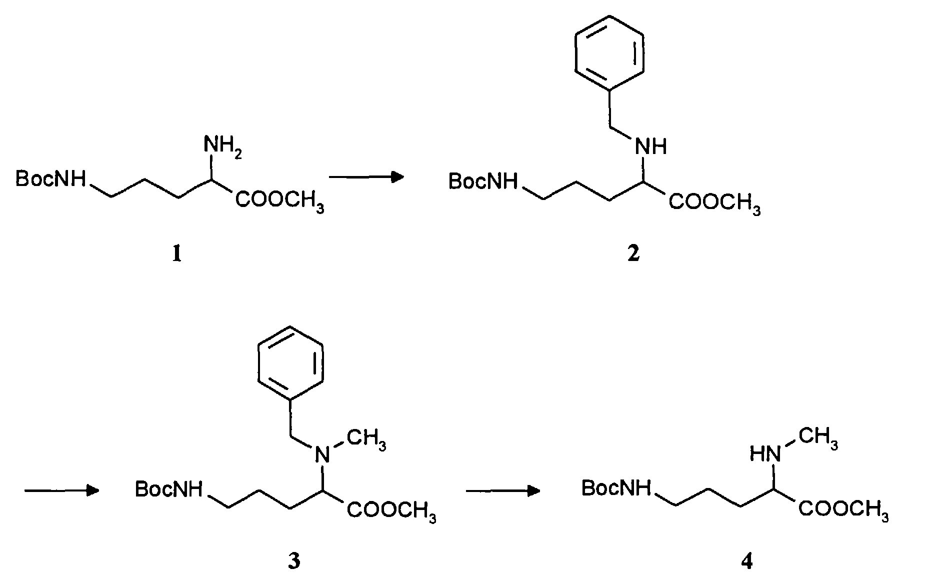

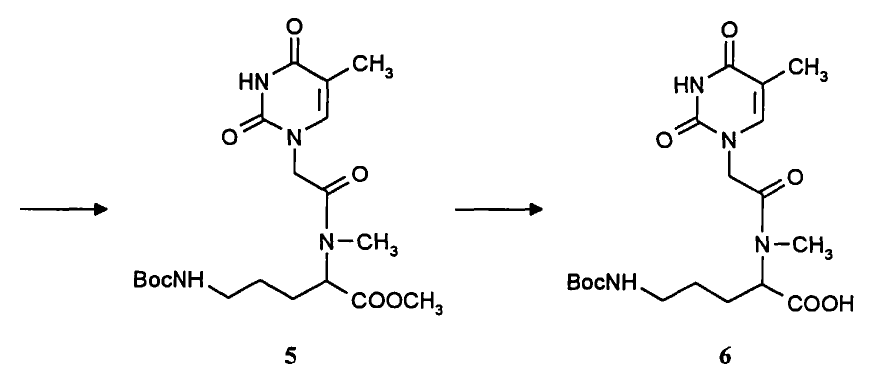

Derivate anderer Nukleobasen und anderer α,ω-Diaminocarbonsäuren, wie z.B. Lysin oder 2,4-Diaminobuttersäure sind analog zugänglich. An Stelle der L-Aminosäurederivate können auch die D-Aminosäurederivate eingesetzt werden. Für das 2,4-Diaminobuttersäurederivat kann die Synthese auch ausgehend von Glutaminsäure erfolgen.

Ausgehend von Fmoc-geschützter L-Glutaminsäure **7** wird durch Umsetzung mit Paraformaldehyd in Gegenwart von p-Toluolsulfonsäure das Oxazolidinon **8** erzeugt. Anschließend wird ein Curtius-Abbau in Gegenwart von Benzylalkohol durchgeführt, so daß man das α-Fmoc-γ-Z-geschützte Oxazolidinon-Derivat **9** erhält. Durch Umsetzung von **9** mit Triethylsilan und TFA wird das α-*N*-methylierte 2,4-Diaminobuttersäurederivat **10** erhalten. Veresterung nach der Cäsiumsalz-Methode mit Methyliodid ergibt **11**. Nach Abspaltung der Fmoc-Schutzgruppe mit Piperidin gefolgt von der Einführung der Nukleobase (z.B. Carboxymethylthymin) erhält man Verbindung **12**. Den zur Festphasensynthese geeigneten Baustein **13** erhält man nach Verseifung des Methylesters und Abspaltung der Z-Schutzgruppe, gefolgt von der Einführung einer Boc-Schutzgruppe.

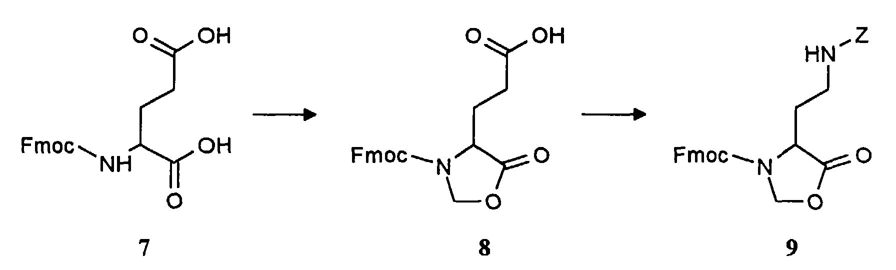

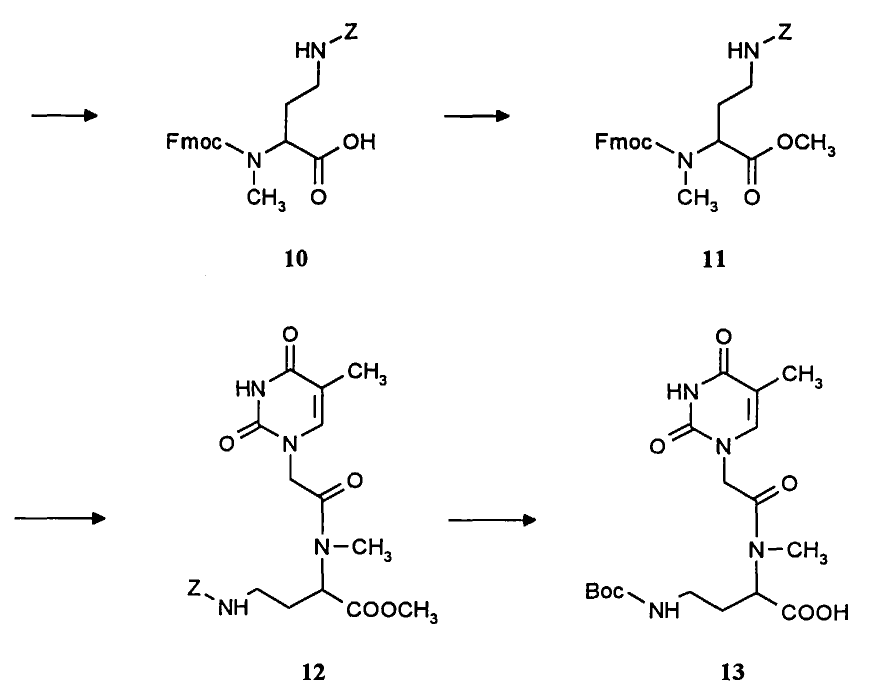

### Oligomerisierung

Die Verknüpfung der Bausteine zu Oligomeren kann in Lösung erfolgen, wird jedoch vorzugsweise mittels Festphasensynthese durchgeführt (siehe: Merrifield, R.B., J. Am. Chem. Soc., 85, (1963, 2149). Hierzu wird bevorzugt ein Peptidsynthesizer, insbesondere das Modell 431-A der Firma Applied Biosystems, eingesetzt. Als polymere Träger stehen verschiedene kommerziell erhältliche Harze zur Verfügung; bevorzugt werden die PAM-, MBHA- und HMP-Resins der Firma Applied Biosystems verwendet. Die Bausteine werden in Analogie zur konventionellen Peptidsynthese durch gezielte Verwendung einer Schutzgruppenstrategie am N-Terminus, bevorzugt unter Nutzung des Fmoc- oder Boc-Verfabrens, verknüpft. Die Aktivierung erfolgt in der Regel in N-Methyl-2-Pyrrolidon (NMP) durch Umsetzung mit Hydroxybenzothazol/Dicyclohexylcarbodiimid, oder aber auch unter Nutzung anderer bekannter Aktivierungsverfahren aus der Peptidchemie (beispielsweise Uroniumsalze, wie TBTU, HBTU, BOP, PyBOP usw. in NMP oder anderen Lösungsmitteln, wie DMF, DMSO oder DCM). Die festphasengebundenen Verbindungen werden im Anschluß an die Oligomerisierung durch spezielle Abspaltreagenzien wie HF oder Trifluormethansulfonsäure (Boc-Methode; PAM- oder MBHA-Resin) oder durch Trifluoressigsäure (Fmoc-Methode; HMP-Resin) abgetrennt und durch Filtration vom polymeren Träger entfernt. Bekannte Übersichtsartikel mit detaillierten Beschreibungen des verwendeten Verfahrens sind beispielsweise a) Barany, G., Kneib-Cordonier, N., Mullen, D.G., Int. J. Pept. Protein Res., 30, 1987, 705ff und b) Fields, G.B. Noble, R.C., Int. J. Pept. Protein Res., 35, 1990, 161-214. Die Reaktionsprodukte werden durch präparative HPLC, insbesondere unter Nutzung der "reversed phase" Methode bei Verwendung von RP 8 Säulen mit einem Lösungsmittelgemisch, wie beispielsweise einem ansteigenden Gradienten von Trifluoressigsäure in Acetonitril oder Acetonitril/Wasser isoliert. Die Charakterisierung der Verbindungen erfolgt insbesondere durch die Massenspektroskopie.

### Hybridisierungseigenschaften

Untersuchungen zu den Hybridisierungseigenschaften, sowie der Nuklease-und Proteasestabilität wurden analog zu den Experimenten in EP 646 595 A1 durchgeführt.

### Experimenteller Teil

### Monomere

### Beispiel 1: δ-N-Boc-α-N-benzyl-L-ornithinmethylester

13,03 g ω-*N*-Boc-L-ornithinmethylesterhydrochlorid werden in 50 ml Methanol gelöst. Zu dieser Lösung wird Molekularsieb (3 Å) und Triethylamin (6,9 ml) gegeben und 30 Min. bei Raumtemperatur gerührt. Anschließend wird Benzaldehyd (4,93 ml) gefolgt von NaBH₃CN (3,19 g) zugegeben und über Nacht bei Raumtemperatur gerührt. Nach beendeter Reaktion wird zur Trockne eingedampft, der Rückstand in Essigsäureethylester aufgenommen und nacheinander mehrmals mit ges. NaHCO₃-Lsg. und ges. NaCl-Lsg. gewaschen. Die organische Phase wird über Na₂SO₄ getrocknet. Danach wird zur Trockne eingedampft und der Rückstand an Kieselgel (Laufmittel: Essigsäureethylester / n-Hexan 1:1) chromatographiert.
- Ausbeute:: 9,13 g
- R_{f}:: 0,47 Laufmittel: Essigsäureethylester / n-Hexan 1:1

### Beispiel 2: δ-N-Boc-α-N-benzyl-α-N-methyl-L-ornithinmethylester

5 g δ-*N*-Boc-α-*N*-benzyl-L-ornithinmethylester werden in 50 ml DMF gelöst. Anschließend werden 2,05 g K₂CO₃ zugegeben. Zu der erhaltenen Suspension wird schließlich das Methyliodid (0,92 ml) langsam zugetropft und über Nacht bei Raumtemperatur gerührt. Danach wird das DMF abdestilliert und der Rückstand in Essigsäureethylester aufgenommen. Die organische Phase wird nacheinander mit 10 %iger Natriumthiosulfatlösung und NaCl-Lsg. gewaschen. Anschließend wird die organische Phase über Na₂SO₄ getrocknet und dann zur Trockne eingedampft.
- Ausbeute:: 4,77 g
- R_{f}:: 0,49 Laufmittel: Essigsäureethylester / n-Hexan 1:2

### Beispiel 3: δ-N-Boc-α-N-methyl-L-ornithinmethylester

4,77 g δ-*N*-Boc-α-*N*-benzyl-α-*N*-methyl-L-ornithinmethylester werden in 150 ml Methanol gelöst. Die Lösung wird mit Palladium auf Kohle (10 %, 1 g) versetzt und es wird über Nacht hydriert. Anschließend wird vom Katalysator abgesaugt und das Filtrat zur Trockne eingeengt.
- Ausbeute:: 3,37 g
- R_{f}:: 0,55 Laufmittel: CH₂Cl₂ / CH₃OH 9:1

### Beispiel 4: δ-N-Boc-α-N-methyl-α-N-(thymin-1-yl)-acetyl-L-ornithinmethylester

6,23 g 1-Carboxymethylthymin werden in 100 ml DMF gelöst und die Lösung auf -30°C gekühlt. Anschließend werden 6,9 g 1-Hydroxy-1H-benzotriazol (HOBt) gefolgt von 8,5 g *N*'-(3-Dimethylaminopropyl)-*N*-ethylcarbodiimid (EDCI) zugegeben. Man läßt weitere 15 Min bei -30°C rühren. Danach werden 3,37 g δ-*N*-Boc-α-*N*-methyl-L-ornithinmethylester gelöst in DMF zugetropft. Nach beendeter Zugabe wird mit Triethylamin ein pH von 9 eingestellt und weitere 60 Min bei -30°C gerührt. Zur Vervollständigung der Reaktion läßt man über Nacht bei Raumtemperatur rühren. Nach dieser Zeit wird zur Trockne eingedampft, der Rückstand in Essigsäureethylester aufgenommen und anschließend die organische Phase nacheinander mit 1 N HCl-Lsg., ges. NaHCO₃-Lsg. und ges. NaCl-Lsg. gewaschen. Die organische Phase wird anschließend über Na₂SO₄ getrocknet und dann zur Trockne eingedampft.
- Ausbeute:: 3,95 g
- R_{f}:: 0,80 Laufmittel: CH₂Cl₂ / CH₃OH / HOAc 80:20:1

### Beispiel 5: δ-N-Boc-α-N-methyl-α-N-(thymin-1-yl)acetyl-L-ornithin

3,95 g δ-*N*-Boc-α-*N*-methyl-α-*N*-(thymin-1-yl)acetyl-L-ornithinmethylester werden in 100 ml Dioxan gelöst und die Lösung wird mit 10,2 ml 1 N NaOH versetzt. Es wird 2 h bei Raumtemperatur gerührt. Nach dieser Zeit werden erneut 10,2 ml 1 N NaOH zugesetzt und über Nacht bei Raumtemperatur gerührt. Anschließend wird das Dioxan abdestilliert und der Rückstand mit Essigsäureethylester und 1 N HCl versetzt. Die sauer gestellte wäßrige Phase wird mehrmals mit Essigsäureethylester nachextrahiert. Die vereinigten organischen Phasen werden über Na₂SO₄ getrocknet und schließlich zur Trockne eingedampft.
- Ausbeute:: 2,9 g
- R_{f}:: 0,31 Laufmittel: CH₂Cl₂ / CH₃OH / HOAc 80:20:1

### Beispiel 6: δ-N-Boc-α-N-methyl-α-N-(N⁴-Z-cytosin-1-yl)acetyl-L-ornithinmethylester

4,69 g *N*^{*4*}-Z-1-Carboxymethylcytosin werden in 100 ml DMF gelöst und mit 3,36 g δ-*N*-Boc-α-*N*-methyl-L-ornithinmethylester analog Beispiel 4 umgesetzt.
- Ausbeute:: 6,5 g
- R_{f}:: 0,53 Laufmittel: CH₂Cl₂ / CH₃OH 9:1

### Beispiel 7: δ-N-Boc-α-N-methyl-α-N-(N⁴-Z-cytosin-1-yl)acetyl-L-ornithin

6,5 g δ-*N*-Boc-α-*N*-methyL-α-*N*-(*N*^{*4*}-Z-cytosin-1-yl)acetyl-L-ornithinmethylester werden in 100 ml Dioxan gelöst und analog Beispiel 5 umgesetzt.
- Ausbeute:: 5,06 g
- R_{f}:: 0,45 Laufmittel: CH₂Cl₂ / CH₃OH / HOAc 80:20:1

### Beispiel 8: α-N-Benzyl-γ-N-Boc-L-2,4-diaminobuttersäuremethylester

γ-*N*-Boc-L-2,4-diaiminobuttersäuremethylester (2,33 g) wird in 40 ml CH₃OH gelöst. Zu dieser Lösung gibt man 1,15 g Benzaldehyd und rührt 15 Min bei Raumtemperatur. Anschließend werden 660 mg Natriumcyanoborhydrid zugegeben und über Nacht bei Raumtemperatur gerührt. Es wird schließlich zur Trockne eingedampft, der Rückstand in Essigsäureethylester aufgenommen und mehrmals mit ges. NaHCO₃-Lsg. und ges. NaCl-Lsg. gewaschen. Nach dem Waschen wird die organische Phase über Na₂SO₄ getrocknet und dann zur Trockne eingeengt. Der Rückstand wird an Kieselgel chromatographiert (Laufmittel: Essigsäureethylester / n-Hexan 1:1).
- Ausbeute:: 700 mg
- R_{f}:: 0,47 Laufmittel: Essigsäureethylester / n-Hexan 1:1

### Beispiel 9: α-N-Benzyl-α-N-methyl-γ-N-Boc-L-2,4-diaminobuttersäuremethylester

α-*N*-Benzyl-γ-*N*-Boc-L-2,4-diaminobuttersäuremethylester (1,56 g) wird in DMF gelöst und mit 701 mg K₂CO₃ sowie 309 µl CH₃I versetzt. Anschließend wird 3 d bei Raumtemperatur gerührt. Nach dieser Zeit wird zur Trockne eingeengt und der Rückstand in Essigsäureethylester aufgenommen. Die organische Phase wird nacheinander mit 10 %iger Natriumthiosulfat-Lsg. und ges. NaCl-Lsg. gewaschen. Nach Phasentrennung wird über Na₂SO₄ getrocknet und das Lösemittel eingedampft.
- Ausbeute:: 1,45 g
- R_{f}:: 0,65 Laufmittel: Essigsäureethylester / n-Hexan 1:1

### Beispiel 10: α-N-Methyl-γ-N-Boc-L-2,4-diaminobuttersäuremethylester

1,45 g α-*N*-Benzyl-α-*N*-methyl-γ-*N*-Boc-L-2,4-diaminobuttersäuremethylester werden in 150 ml CH₃OH gelöst und an Pd/C (10 %) hydriert. Nach beendeter Reaktion wird vom Katalysator abfiltriert und das Filtrat zur Trockne eingedampft.
- Ausbeute:: 1,0 g
- R_{f}:: 0,51 Laufmittel: CH₂Cl₂ / CH₃OH 9:1

### Beispiel 11: α-N-Methyl-α-N-(thymin-1-yl)acetyl-γ-N-Boc-L-2,4-diaminobuttersäuremethylester

1,1 g α-*N*-Methyl-γ-*N*-Boc-L-2,4-diaminobuttersäuremethylester werden analog Beispiel 4 mit 2,1 g 1-Carboxymethylthymin, 2,05 g HOBt und 2,9 g EDCI umgesetzt.
- Ausbeute:: 1,53 g
- R_{f}:: 0,55 Laufmittel: CH₂Cl₂ / CH₃OH 9:1

### Beispiel 12: α-N-Methyl-α-N-(thymin-1-yl)acetyl-γ-N-Boc-L-2,4-diaminobuttersäure

1,5 g α-*N*-Methyl-α-*N*-(thymin-1-yl)acetyl-γ-*N*-Boc-L-2,4-diaminobuttersäuremethylester werden analog Beispiel 5 durch Umsetzung mit 1 N NaOH verseift.
- Ausbeute:: 300 mg
- R_{f}:: 0,28 Laufmittel: CH₂Cl₂ / CH₃OH / HOAc 80:20:1

### Beispiel 13: α-N-Fmoc-4-(N-Z-2-amino-ethyl)-oxazolidin-5-on

Fmoc-L-Glutaminsäure (9,22 g) wird in 500 ml Toluol vorgelegt, mit Paraformaldehyd (4,85 g) und p-Toluolsulfonsäure (485 mg) versetzt. Anschließend wird 2,5 h unter Rückfluß am Wasserabscheider gekocht. Nach dieser Zeit läßt man auf Raumtemperatur abkühlen und gibt Benzylalkohol (3 g), Triethylamin (3,8 ml) und Diphenylphosphorylazid (5,9 ml) zu. Die Lösung wird schließlich für 4 h auf 90 bis 95°C erhitzt. Danach wird die Toluolphase nacheinander mehrmals mit Citronensäure-Lösung, ges. NaHCO₃-Lsg., Wasser und ges. NaCl-Lsg. gewaschen. Es wird über Na₂SO₄ getrocknet und anschließend zur Trockne eingedampft. Das Produkt wird aus Essigsäureethylester / n-Hexan 1:1 ausgefällt.
- Ausbeute:: 3,61 g
- R_{f}:: 0,49 Laufmittel: Essigsäureethylester / n-Hexan 1:1

### Beispiel 14: α-N-Fmoc-α-N-methyl-γ-N-Z-L-2,4-diaminobuttersäure

14,5 g α-*N*-Fmoc-4-(*N*-Z-2-aminoethyl)-oxazolidin-5-on werden in 150 ml CH₂Cl₂ gelöst. Zu dieser Lösung gibt man 10,3 g Triethylsilan sowie 150 ml TFA und läßt 18 h bei Raumtemperatur rühren. Anschließend wird zur Trockne eingedampft und der Rückstand mehrmals mit Toluol codestilliert. Zur Reinigung wird der Rückstand schließlich an Kieselgel mit CH₂Cl₂ / CH₃OH 4:1 als Laufmittel säulenchromatographiert.
- Ausbeute:: 8,8 g
- R_{f}:: 0,41 Laufmittel: CH₂Cl₂ / CH₃OH 4:1

### Beispiel 15: α-N-Fmoc-α-N-methyl-γ-N-Z-L-2,4-diaminobuttersäuremethylester

530 mg α-*N*-Fmoc-α-*N*-methyl-γ-*N*-Z-L-2,4-diaminobuttersäure wird in einem Gemisch aus Wasser / Ethanol (1 : 1) gelöst und mit 200 mg Cs₂CO₃ versetzt. Anschließend wird 1 h bei Raumtemperatur gerührt. Nach dieser Zeit wird zur Trockne eingedampft und mehrmals mit Toluol codestilliert. Der Rückstand wird schließlich in DMF gelöst und die Lösung mit 155 mg Methyliodid versetzt. Es wird schließlich über Nacht bei Raumtemperatur gerührt. Nach beendeter Reaktion wird zur Trockne eingedampft und der Rückstand mehrmals mit Toluol codestilliert. Anschließend wird in Essigsäureethylester aufgenommen und die organische Phase nacheinander mit ges. NaCl-Lsg., ges. NaHCO₃-Lsg. und erneut mit ges. NaCl-Lsg. gewaschen. Die organische Phase wird über Na₂SO₄ getrocknet und danach zur Trockne eingeengt. Zur Reinigung wird der Rückstand an Kieselgel chromatographiert (Laufmittel: Essigsäureethylester / n-Hexan 1:1).
- Ausbeute:: 260 mg
- R_{f}:: 0,57 Laufmittel: Essigsäureethylester / n-Hexan 1:1

### Beispiel 16: α-N-Methyl-γ-N-Z-L-2,4-diaminobuttersäuremethylester

500 mg α-*N*-Fmoc-α-*N*-methyl-γ-*N*-Z-L-2,4-diaminobuttersäuremethylester werden in DMF gelöst und mit 0,15 ml Piperidin 10 Min bei Raumtemperatur behandelt. Anschließend wird zur Trockne eingedampft, der Rückstand in CH₂Cl₂ aufgenommen und die organische Phase mit Wasser gewaschen. Nach der Phasentrennung wird die organische Phase über Na₂SO₄ getrocknet und zur Trockne eingedampft. Der Rückstand wird zur Reinigung an Kieselgel (Laufmittel: CH₂Cl₂ / CH₃OH 10:1) chromatographiert.
- Ausbeute:: 216 mg
- R_{f}:: 0,58 Laufmittel: CH₂Cl₂ / CH₃OH 10:1

### Beispiel 17: γ-N-Z-α-N-methyl-α-N-(thymin-1-yl)acetyl-L-2,4-diaminobuttersäuremethylester

200 mg γ-Z-α-*N*-Methyl-L-2,4-diaminobuttersäuremethylester werden analog Beispiel 4 mit 1-Carboxymethylthymin, EDCI und HOBt in Gegenwart von Triethylamin umgesetzt. Der nach der Aufarbeitung erhaltene Rückstand wird zur Reinigung an Kieselgel (Laufmittel: CH₂Cl₂ / CH₃OH 10:1) chromatographiert.
- Ausbeute:: 290 mg
- R_{f}:: 0,49 Laufmittel: CH₂Cl₂ / CH₃OH 10:1

### Beispiel 18: δ-N-Boc-α-N-methyl-α-N-(N⁶-Z-adenin-9-yl)acetyl-L-ornithinmethylester

0,8 g *N*^{*6*}-Z-Carboxymethyladenin werden in 50 ml DMF gelöst und mit 1,1 g δ-*N*-Boc-α-*N*-methyl-L-ornithinmethylester analog Beispiel 4 umgesetzt.
- Ausbeute:: 1,53 g
- R_{f}:: 0,62 Laufmittel: CH₂Cl₂/CH₃OH 10:1

### Beispiel 19: δ-N-Boc-α-N-methyl-α-N-(N⁶-Z-adenin-9-yl)acetyl-L-ornithin

1,53 g δ-*N*-Boc-α-*N*-methyl-α-*N*-(*N*^{*6*}-Z-adenin-9-yl)acetyl-L-ornithinmethylester werden in 50 ml Dioxan gelöst und analog Beispiel 5 umgesetzt.
- Ausbeute:: 0,96 g
- R_{f}:: 0,41 Laufmittel CH₂Cl₂/CH₃OH/HOAc 80:20:1

### Beispiel 20: δ-N-Boc-α-N-methyl-α-N-(O⁶-benzyl-N²-Z-guanin-9-yl)acetyl-L-ornithinmethylester

0,2 g *O*^{*6*}-Benzyl-*N*^{*2*}-Z-9-carboxymethylguanin werden in 5 ml DMF gelöst und mit 1,3 eq. δ-*N*-Boc-α-*N*-methyl-L-ornithinmethylester gefolgt von jeweils 1,25 eq. Benzotriazol-1-yl-oxy-tris-(dimethylamino)-phosphoniumchlorid (BOPCl) und HOBt sowie 1,3 eq. *N*-Ethyldiisopropylamin versetzt. Nach 3,5 h Rühren bei Raumtemperatur wird das Lösungsmittel entfernt. Das verbleibende Öl wird zwischen Essigsäureethylester und Natriumchloridlösung verteilt. Die organische Phase wird abgetrennt, die wäßrige noch mehrfach mit Essigsäureethylester extrahiert. Die vereinigten organischen Phasen werden mit 0,1 N HCl, gesättigter NaHCO₃-Lösung und NaCl-Lösung gewaschen. Nach Trocknen über Natriumsulfat verbleibt ein Rückstand, der an Kieselgel chromatographiert wird (Laufmittel: CH₂Cl₂/CH₃OH 20:1)
- Ausbeute:: 197 mg
- R_{f}:: 0,64 Laufmittel: CH₂Cl₂/CH₃OH 10:1

### Beispiel 21: δ-N-Boc-α-N-methyl-α-N-(O⁶-benzyl-N²-Z-guanin-9-yl)acetyl-L-ornithin

172 mg δ-*N*-Boc-α-*N*-methyl-α-*N*-(*O*^{*6*}-benzyl-*N*^{*2*}-Z-guanin-9-yl)acetyl-L-ornithinmethylester werden in 10 ml Dioxan gelöst und analog Beispiel 5 umgesetzt.
- Ausbeute:: 145 mg
- R_{f}:: 0,72 Laufmittel: CH₂Cl₂/CH₃OH 1:1

### Oligomere

### Beispiel 22: Festphasensynthese von NH2-(T1-T2)6-Lys-NH2

- T1 =: Aminoethylglycin-Thymin-Baustein, gemäß WO 92/20703
- T2 =: δ-*N*-Boc-α-*N*-methyl-α-*N*-(thymin-1-yl)acetyl-L-ornithin (z.B. aus Bsp. 5)

Die Oligomersierung erfolgt unter Nutzung einer leicht abgeänderten Version des im Peptidsynthesizer ABI 431-A vorhandenen Programmes für Boc-Small-Scale-Reaktionen.

32,5 mg (0,025 mmol) MBHA-Resin (Belegung: 0,77 mmol/g) werden im Reaktionsgefäß vorgelegt. Der Träger wird mit Diisopropylethylamin neutralisiert und mit DCM gewaschen. Die Aktivierung von 1 mmol Boc-Lys(2-chloro-Z)-OH (0,41 g, Applied Biosystems) und jeweils 52 mg T2-Baustein bzw. 48 mg T1-Baustein erfolgt durch Umsetzung mit 135 mg (1,0 mmol) Hydroxybenzotriazol bzw. einer entsprechenden 1:1 Mischung aus Hydroxybenzotriazol und Hydroxyazabenzotriazol (Perseptive Biosystems) und 206 mg (1,0 mmol) Dicyclohexylcarbodiimid in N-Methyl-2-pyrrolidon (NMP). Das Hydroxy(aza)benzotriazol wird dabei zur besseren Löslichkeit der Bausteine zusammen mit NMP in der die Aminosäure enthaltenen Kartusche vorgelegt. Die tert.-Butyloxycarbonyl-Schutzgruppe des trägergebundenen Zwischenproduktes wird vor jedem Kupplungsschritt durch Behandlung mit Trifluoressigsäure abgespalten und der Träger anschließend mit Diisopropylethylamin neutralisiert und mit DCM gewaschen. Im Anschluß erfolgt die schrittweise Ankopplung an den polymeren Träger. Nach der letzten Kopplung wird die Boc-Schutzgruppe durch Behandlung mit Trifluoressigsäure entfernt. Wiegen des getrockneten Trägers ergibt 42,0 mg gebundenes Peptid. Die Abspaltung des Oligomeren vom Träger erfolgt durch 2-stündige Behandlung des Polymeren mit 4,5 ml HF und 0,5 ml Anisol bei 0°C im Teflonkolben. Nach der HF-Abspaltung wird der Rückstand 4 mal mit 15 ml absolutem Diethylether gerührt, um anhaftendes Anisol herauszulösen. Nach jeweils 15 Minuten wird der Ether vorsichtig abdekantiert. Nun wird das Oligomer mit 60 ml 30%iger Essigsäure (4x15 mi, je 15 Minuten lang) extrahiert, die Lösung vom Polymer über eine D3-Fritte getrennt und das Filtrat lyophilisiert.

Man erhält 38,0 mg Rohprodukt, welches zur Reindarstellung mittels RP-HPLC aufgearbeitet wird. Als stationäres Trennmedium verwendet man eine "Eurosil Bioselect 300 A (5 µm)" Säule unter Nutzung des nachfolgend beschriebenen Elutionssystems:
- Eluent A:: 0,1% TFA in Wasser
- Eluent B:: 0,1% TFA in Wasser/Acetonitril (3/7)

Der Gradient wird wie folgt eingestellt:

| Gradient (Min.) | Eluent A (%) | Eluent B (%) |
|---|---|---|
| 0,00 | 95 | 05 |
| 30,00 | 40 | 60 |
| 40,00 | 20 | 80 |
| 45,00 | 20 | 80 |
| 50,00 | 95 | 05 |

Die Detektion erfolgt bei 260 nm mittels eines UV-Detektors. Die Retentionszeit der Zielsubstanz beträgt 21,5 Minuten.

Die aufgereinigte PNA wird nach der HPLC lyophilisiert. Man erhält eine Ausbeute an hochreinem Produkt von 8,2 mg (0,0023 mmol; 9,2 % bezogen auf die theor. mögliche Menge; 19,5 % bezogen auf die tatsächlich harzgebundene Menge). Die Charakterisierung der PNA erfolgt über Massenspektroskopie (LDI-Methode). Das theoretische Molekulargewicht beträgt 3509,5 g/mol, gemessen werden 3508,6 g/mol.

### Bestimmung der Annealing Temperatur:

Der entsprechenden DNA-Gegenstrang (A12) wird auf einem ABI 380B DNA-Synthesizer mit Hilfe der Phosphoramiditmethode nach dem small scale Cycle des Herstellers Applied Biosystems hergestellt.

Die Bestimmung der Annealing Temperatur erfolgte unter Nutzung eines Perkin Elmer "Lambda Bio" UV-Vis-Spektrometers unter Verwendung der vom Hersteller angegebenen Methode "PE-TEMP".

Hierzu wird soviel PNA des oben erwähnten Beispiels in 700 µl Wasser gelöst, bis sich eine Absorbtion von 0,3 ergibt. Desgleichen erfolgt mit dem korrespondierenden A12-DNA-Strang. Anschließend werden beide Stränge vereint und das Wasservolumen auf 1,5 ml erhöht. Die vereinigten Stränge werden nun 5 Minuten lang bei 95°C erhitzt und dann über Nacht in einem Styroporgefäß langsam abgekühlt. Anschließend werden die Doppelstränge gemaß der Methode "PE-TEMP" im Temperaturbereich von 20°C - 80°C auf ihre Absorbtion hin untersucht. Der Umkehrpunkt (Maximum der 1. Ableitung) der entstehenden Absorptionskurve entspricht dann der gemessenen Annealing-Temperatur auf der Temperaturskala.

Mit Hilfe dieser Methode kann für die DNA-PNA Hybridisierung des oben erwähnten Beispiels eine Annealing-Temperatur von 41°C festgestellt werden.

### Nachweis von DNA-Einzelstrangbindung durch Gel-shift-Analysen

### Beispiel 23

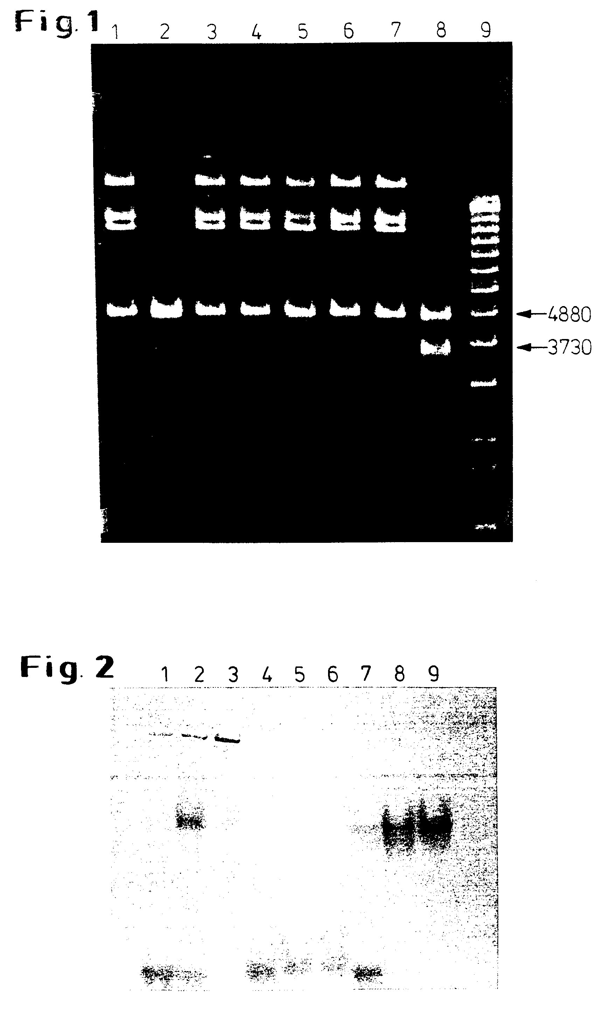

1 µg Oligonukleotid von entsprechender Basensequenz wird mit Polynukleotidkinase und γ-ATP am 5'-Ende in bekannter Weise in einem Volumen von 10 µl markiert (Sambrook, Fritsch, Maniatis: Molecular Cloning, A Laboratory Manual, Cold Spring Harbor, 1989). Nach der Markierung wird die Probe zur Denaturierung des Enzyms bei 70°C für 10 min erhitzt und anschließend mit 9 µg nicht markiertem Oligomer vermischt. 1 µl dieses Ansatzes wird mit einer gewünschten Menge zu testendem Nukleinsäure-bindenden Oligomer versetzt (1-10 µg) und in einem Volumen von 20 µl 30 min bei 22°C (Raumtemperatur) inkubiert (Hybridisierung). Danach wird die Probe 30 min auf Eis gestellt. Ein nicht hybridisiertes markiertes Oligomer wird in gleicher Weise behandelt und dient als Kontrolle. Die Proben werden auf ein 15 % Polyacrylamidgel mit 1x Tris-Borat-EDTA-Puffer aufgetragen. Das Gel und der Puffer wurden im Kühlschrank (8°C) vorgekühlt, die Elektrophorese wurde über Nacht mit 55 V im Kühlschrank laufen gelassen. Nach der Elektrophorese wurde ein Autoradiogramm auf AGFA-Film erstellt (Exponierzeit 16 Stunden, Fig. 1).

### Legende zu Fig. 1:

- 1:: 1 µg P-33-markiertes poly-A, 12-mer
- 2:: 1 µg P-33-markiertes poly-A, 12-mer + 1 µg (I)
- 3:: 1 µg P-33-markiertes poly-A, 12-mer + 5 µg (I)
- 4:: 1 µg P-33-markiertes poly-A, 12-mer + 1 µg (II)
- 5:: 1 µg P-33-markiertes poly-A, 12-mer + 5 µg (II)
- 6:: 1 µg P-33-markiertes poly-A, 12-mer + 10 µg (II)
- 7:: 1 µg P-33-markiertes poly-A, 12-mer + 1 µg aus Beispiel 18
- 8:: 1 µg P-33-markiertes poly-A, 12-mer + 5 µg aus Beispiel 18
- 9:: 1 µg P-33-markiertes poly-A, 12-mer + 10 µg aus Beispiel 18
- (I) =: positive Kontrollsubstanz folgender Zusammensetzung (12-mer): NH₂-(T_{E}-T_{P}-T_{E}-T_{P}-T_{E}-Glycin-T_{Y})₂- 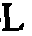 -Lysin-NH₂ mit T_{E} = Aminoethylglycin-Thymidintyp, T_{P} = 4-R-Amino- 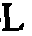 -prolin-Thymidintyp und T_{Y} = 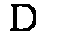 -trans-Pyrolidincarbonsäure-Thymidintyp
- (II) =: negative Kontrollsubstanz folgender Zusammensetzung (12-mer): NH₂-(T_{E}-T_{E}-Glycin-T_{B})₄- 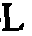 -Lysin-NH₂ mit T_{B} = 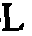 -Aminobuttersäure-Thymidintyp

### Ergebnisse:

Spur 1 aus Fig. 1 markiert die Position eines nicht geshifteten Derivates. Die Spuren 2 und 3 zeigen eine Titration mit einer positiv-shiftenden Verbindung. Die Spuren 4 - 6 sind Tirationen mit einer nicht shiftenden Verbindung. Die Spuren 7 - 9 zeigen die deutlichen Shifts der erfindungsgemäßen Verbindung aus Beispiel 18.

### Nachweis der Strangverdrängung in doppelsträngiger Plasmid-DNA

### Beispiel 24

Nachfolgend wird der Test zum experimentellen Nachweis der DNA Doppelstrangverdrängung durch den PNA-Typ des Beispiels 18 beschrieben. Diese Eigenschaft der DNA Doppelstrangverdrängung ist mit Ribosephosphat-, Ribosemethylphosphonat-, Ribosephosphorothioat-Verknüpfung von Nukleobasen und anderen nukleinsäure-ähnlichen Backbonetypen nicht zu erreichen und stellt daher eine besondere biologische Wirksamkeit des PNA-Typs aus Beispiel 18 dar.

Die im Beispiel eingesetzte Plasmid-DNA ist ein Modellsubstrat für den Nachweis der DNA Doppelstrangverdrängung. Andere Plasmide, die entsprechende Zielsequenzen mit komplementären Nukleobasensequenz zu den zu prüfenden PNA aus Beispiel 18 enthalten, sind in gleicher Weise für den Test verwendbar. Dies laßt den Schluß zu, daß Nukleobasensequenzen aus krankheitsbedingenden Genen, die komplementär sind zum PNA-Typ aus Beispiel 18 von diesem PNA-Typ gebunden werden und dadurch das Gen inaktivieren können.

In diesem Beispiel wird doppelsträngige, zirkuläre Plasmid-DNA von 4880 Basenpaaren Länge, die zwei poly-Adenin Sequenzbereiche mit mindestens neun aufeinanderfolgenden Adeninnukleotiden im Abstand von 1150 Basenpaaren enthält, in den hier beschriebenen Tests verwendet.

Acht parallel angesetzte Proben, bezeichnet (1 - 8), enthielten je 0,5 µg ungeschnittene Plasmid-DNA in 14 µl H₂O. Den Proben 2 - 8 wurden je 1,0 µl Lösung mit je 1,0 µg Testsubstanzen zugesetzt. Die Probe 2 (siehe Fig. 2) enthielt 1,0 µg PNA-Typ aus Beispiel 18. Die Probe 8 enthielt ein 10mer 2-Aminoethylglycinthymin-PNA-Typ als Positivkontrolle. Alle anderen Proben (1, 3 - 7) des Testansatzes, der in der Fig. 2 dokumentiert ist, enthielten andere Testsubstanzen. Die Probe 1 diente als Negativkontrolle.

Die Proben wurden in geschlossenen Eppendorfreaktionsgefäßen 45 min bei 37°C inkubiert. Anschließend wurde in alle Proben 4 µl Puffer (250 mM Na-Acetat, 1 M NaCl, 2,5% Glycerin, 5 mM ZnCl₂, pH 4,4) und je 1,0 µl S1-Nuclease aus Aspergillus oryzae, (Fa. Boehringer Mannheim) mit einer Aktivität von 10 U/ml gegeben. Nach einer Inkubation von 15 min. bei 30°C wurden die Proben auf Eis gesetzt, 1 µl 0,5 M EDTA und 3 µl Auftragspuffer (50% Gycerin, 0,25% Bromphenolblau in 40 mM Tris-HCl, 20 mM Natriumacetat, 1 mM EDTA, pH = 7,2) zugegeben und ohne Zeitverzug die Proben über 1,2% Agarosegele elektrophoretisch aufgetrennt und nach Anfärbung mit Ethidiumbromid die Größe der entstandenen Plasmid-Fragmente im Gel im Vergleich zu einem Molekulargewichts-Standard (1 kB Leiter, Fa. GIBCO-BRL, D-76339 Eggenstein, Probe 9 in Fig. 2) auf dem Transilluminator bei 254 nm UV-Licht bestimmt.

Es zeigte sich, daß in der Probe 2 der PNA-Typ aus Beispiel 18 mit einer Konzentration von 1,3 · 10⁻⁵ M unter diesen Bedingungen die verdrillten, zirkulären Plamide mit einem apparenten Molekulargewicht größer 4880 Basenpaaren (s. obere Banden in der Fig. 2) bindet und Doppelstrangverdrängung bewirkte. DNA-Fragmente der Größe 4880 Basenpaare (Linearisierung), 3730 Basenpaare und 1150 Basenpaare (letzteres Fragment ist unter diesen Bedingungen in Fig. 2 kaum sichtbar) werden durch die S1-Nuklease-Reaktion gebildet. Durch diese spezifisch gebildeten DNA-Fragmente wird die sequenzselektive Bindung des PNA-Typs aus Beispiel 18 an doppelsträngige DNA nachgewiesen. Ein vergleichbares Reaktionsergebnis ist in Probe 8 mit der Positivkontrolle, einem 2-Aminoethylgylcyl-PNA-Typ, erhalten worden (Fig. 2). Für 2-Aminoethylgylcyl-PNA wurde die Sequenzselektive DNA-Doppelstrangverdrängung in der Literatur nachgewiesen (Vadim Demidov et al., Nucleic Acids Research 21, 2103 (1993)).

Mit einem modifizierten Testansatz, bei dem in die Proben anstelle der zirkulären, ungeschnittenen Plasmid-DNA eine Plasmid-DNA gegeben wurde, die durch Restriktionsendonukleaseverdau mit dem Enzym Xba I (Fa. Boehringer Mannheim) linearisiert war, entstanden ebenfalls in den Testansätzen 2 und 8 DNA-Fragmente der Länge 3730 und 1150, die durch die sequenzselektive Bindung der eingesetzten PNA-Typen erklärt werden können.

Weiterhin wurde auch bei höherer Salzkonzentration mit 5 mM Tris HCl, 1 mM MgCl₂, 10 mM NaCl, pH 7,0 anstelle von Wasser DNA Doppelstrangverdrängung mit dem PNA-Typ aus Beispiel 18 an doppelsträngiger Plasmid-DNA nachgewiesen.

Mit diesen Testreihen war die sequenzselektive Bindung des PNA-Typ aus Beispiel 18 an doppelsträngige DNA und der Nachweis der dadurch entstehenden Einzelstrang-DNA durch S1-Nukleaseverdau nachweisbar.

### Legende zu Fig. 2:

Foto eines Agarosegels, das mit Ethidiumbromid gefärbt wurde und die Testansätze (s.o.) enthält. Mit diesem Elektrophoresegel wurden die DNA-Fragmente nachgewiesen, die durch die Doppelstrangverdrängung des PNA-Typs aus Beispiel 18 an Plasmid-DNA in Gegenwart von S1-Nuklease entstehen. Die Elektrophorese-Spuren 1 - 9 enthalten folgende Proben und Testansätze:
- 1 - 8:: 0,5 µg ungeschnittene Plasmid-DNA (Substrat für jeden Testansatz identisch) und 10 U S1-Nuklease
- 1:: Negativkontrolle, die zugesetzte Testsubstanz bewirkt keine Doppelstrangverdrängung
- 2:: Zusatz von 1,0 µg PNA-Typ aus Beispiel 18
- 3 - 7:: Zusatz von anderen Testsubstanzen, die nicht dem PNA-Typ aus Beispiel 18 entsprechen und zu keiner Doppelstrangverdrängung führen
- 8:: Zusatz von 1,0 µg 2-Aminoethylglycyl-PNA als Positivkontrolle führt in gleicher Weise zum Nachweis der Doppelstrangverdrängung durch das Auftreten des Fragments bei 3730 Basenpaaren wie die Testsubstanz im Ansatz 2 mit dem PNA-Typ aus Beispiel 18.
- 9:: Molekulargewichtsstandard (1 kB Leiter, Fa. GIBCO-BRL)

## Patentansprüche

1. Verbindungen der allgemeinen Formel (I) 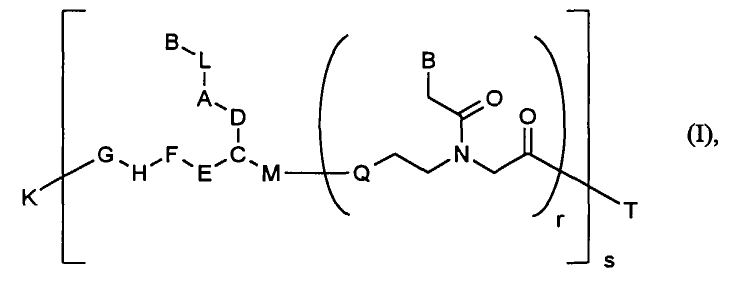 in der
A für -CO-, -CHR oder -CRR'- steht,
B für alle natürlichen oder unnatürlichen Nukleobasen, wie z.B. Thymin, Uracil, Cytosin, Adenin, Guanin, Hypoxanthin oder durch chemische Modifikation von diesen abgeleitete Derivate oder halogenierte Vorstufen von diesen, gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert, steht,
C für -CH- oder -CR- steht, wobei C einheitlich S oder R konfiguriert sein kann,
D für -NH-, -NR-, -CH₂-, -CHR- oder -CRR'- steht,
E für -NH-, -NR-, -CH₂-, -CHR, -CRR'-, -O- oder -S- steht,
A und E miteinander über eine Alkylkette [(-CH₂)ₙ-, mit n = 0, 1, 2] verbunden sein können,
F für -CH₂-, -CO-, SO₂-,-SO- oder -CS- steht,
G für -NH-, -NR-, -O- oder -S- steht,
H für eine Bindung, -CH₂-CH₂- oder -CR¹R²- steht, worin
R¹ für Wasserstoff oder Methyl steht und
R² für Wasserstoff, gegebenenfalls durch Amino oder Hydroxy substituiertes C₁-C₄-Alkyl oder für Mercaptomethyl, Methylthioethyl, Carboxymethyl, Carboxyethyl, Carbamoylmethyl, Carbamoylethyl, Guanidinopropyl oder für gegebenenfalls durch Amino, Nitro, Halogen, Hydroxy oder Methoxy substituiertes Phenyl oder Benzyl oder für Naphthylmethyl, Indolylmethyl, Imidazolylmethyl, Triazolylmethyl oder Pyridylmethyl steht, wobei funktionelle Gruppen gegebenenfalls geschützt vorliegen und die Gruppe -CR¹R²-stereochemisch einheitlich in der L-Form oder D-Form konfiguriert sein kann,
G und H miteinander über eine Alkylkette [(-CH₂)ₙ- mit n = 3 oder 4] verbunden sein können,
L für (-CH₂)ₚ- mit p = 0, 1 oder 2, -CHR- oder -CRR'- steht,
M für -CH₂-, -CO-, -SO₂-, -SO- oder -CS- steht,
Q für NH, O, S oder NR³ steht, worin
R³ für C₁-C₈-Alkyl, C₂-C₈-Alkenyl, Phenyl, Pyridyl, Benzyl oder Naphthylmethyl steht.
K für Wasserstoff, Carrier-System, löslichkeitsvermittelnde Gruppe oder Acylrest einer natürlichen oder unnatürlichen Aminosäure (C-verknüpfte Aminosäure) steht,
T für Hydroxy, Carrier-System, löslichkeitsvermittelnde Gruppe oder amidisch (N-) verknüpfte natürliche oder unnatürliche Aminosäure steht,
R und R' jeweils unabhängig voneinander für OH, Methyl, Ethyl, n-Propyl, iso-Propyl, n-Butyl, iso-Butyl, sec.-Butyl oder tert.-Butyl, Aralkyl wie beispielsweise Benzyl, α-Naphthylmethyl oder β-Naphthylmethyl oder gegebenenfalls durch Methyl, Halogen oder NO₂ substituiertes Aryl oder Hetaryl wie beispielsweise Phenyl, 2-Pyridyl oder 4-Pyridyl steht,
r für 0 oder 1 steht und
s für einen Wert von 1 bis 30 steht.

2. Verbindungen der allgemeinen Formel (I) gemäß Anspruch 1, worin
A für -CO-, -CHR- oder -CRR'- steht
B für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert sind steht,
C für -CH- oder -CR-, wobei C einheitlich S oder R konfiguriert sein kann steht,
D für -NH-, -NR-, -CH₂-, -CHR- oder -CRR'- steht,
E für -NH-, -NR-, -CH₂-, -CHR-, -CRR'- oder -O-steht,
A und E miteinander über eine Alkylkette [(-CH₂)ₙ-, mit n = 0, 1, 2] verbunden sein kann,
F für -CH₂-, -CO- oder -CS- steht,
G für -NH-, -NR- oder -O- steht,
H für eine Bindung, -CH₂-CH₂- oder -CR¹R²- steht, worin
R¹ für Wasserstoff oder Methyl steht und
R² für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl, 4-Methoxybenzyl, 2-Nitrobenzyl, 3-Nitrobenzyl, 4-Nitrobenzyl, 2-Aminobenzyl, 3-Aminobenzyl, 4-Aminobenzyl, 3,4-Dichlorbenzyl, 4-Iodbenzyl, α-Naphthylmethyl, β-Naphthylmethyl, 3-Indolylmethyl, 4-Imidazolylmethyl, 1,2,3-Triazol-1-yl-methyl, 1,2,4-Triazol-1-yl-methyl, 2-Pyridylmethyl oder 4-Pyridylmethyl steht, wobei funktionelle Gruppen gegebenenfalls geschützt vorliegen und die Gruppe -CR¹R²- stereochemisch einheitlich in der L-Form oder D-Form konfiguriert ist,
G und H miteinander über eine Alkylkette [(-CH₂)ₙ- mit n = 3 oder 4] verbunden sein können,
L für (-CH₂)ₚ- mit p = 0, 1 oder 2, -CHR- oder -CRR'- steht,
M für -CH₂-, -CO-, oder -CS- steht,
Q für NH, O oder NR³ steht, worin
R³ für C₁-C₄-Alkyl, C₂-C₄-Alkenyl, Phenyl, 2-Pyridyl, 4-Pyridyl, Benzyl, α-Naphthylmethyl oder β-Naphthylmethyl steht,
K für Wasserstoff, Carrier-System, löslichkeitsvermittelnde Gruppe oder Acylrest einer natürlichen oder unnatürlichen Aminosäure (C-verknüpfte Aminosäure) steht,
T für Hydroxy, Carrier-System, löslichkeitsvermittelnde Gruppe oder amidisch (N-) verknüpfte natürliche oder unnatürliche Aminosäure steht,
R und R' jeweils unabhängig voneinander für OH, Methyl, Ethyl, n-Propyl oder n-Butyl, Benzyl oder gegebenenfalls durch Methyl, Fluor, Chlor, Brom oder NO₂ substituiertes Phenyl stehen,
r für 0 oder 1 steht und
s für einen Wert von 1 bis 20 steht.

3. Verbindungen der allgemeinen Formel (I) gemäß Anspruch 1, worin
A für -CO-, -CHR- oder -CRR'- steht,
B für alle natürlichen Nukleobasen, wie z.B. Thymin, Uracil, Cytosin, Adenin, Guanin oder Hypoxanthin oder halogenierte Vorstufen von diesen, die gegebenenfalls an den Aminogruppen durch Schutzgruppen wie Acetyl, Trifluoracetyl, Trichloracetyl, Benzoyl, Phenylacetyl, Benzyloxycarbonyl, tert.-Butyloxycarbonyl, Allyloxycarbonyl, (9-Fluorenyl)methoxycarbonyl oder andere in der Peptid- und Nukleinsäurechemie übliche Schutzgruppen substituiert sind steht,
C für -CH-, -CR-, wobei C einheitlich S oder R konfiguriert sein kann steht,
D für -NH-, -N(CH₃)-, -CH₂-, -CHR- oder -CRR'- steht,
E für -NH-, -NR-, -CH₂- oder -CHR- steht,
F für -CH₂-, -CO- oder -CS- steht,
G für -NH- oder -N(CH₃)- steht,
H für eine Bindung, -CH₂-CH₂- oder -CR¹R²- steht, worin
R¹ für Wasserstoff oder Methyl steht und
R² für Wasserstoff, Methyl, iso-Propyl, iso-Butyl, sec.-Butyl, Hydroxymethyl, 1-Hydroxyethyl, Mercaptomethyl, 2-Methylthioethyl, 3-Aminopropyl, 4-Aminobutyl, Carboxymethyl, 2-Carboxyethyl, Carbamoylmethyl, 2-Carbamoylethyl, 3-Guanidinopropyl, Phenyl, Benzyl, 4-Hydroxybenzyl, wobei funktionelle Gruppen gegebenenfalls geschützt vorliegen und die Gruppe -CR¹R²- stereochemisch einheitlich in der L-Form oder D-Form konfiguriert ist,
L für (-CH₂)ₚ- mit p = 0, 1 oder 2, -CHR- oder -CRR'- steht,
M für -CH₂-, -CO-, oder -CS- steht,
Q für NH, O oder NR³ steht, worin
R³ für Methyl, Ethyl, n-Propyl, iso-Propyl, n-Butyl, iso-Butyl, sec.-Butyl, tert.-Butyl, Vinyl, Allyl, Phenyl, 2-Pyridyl, 4-Pyridyl, Benzyl, α-Naphthylmethyl oder β-Naphthylmethyl steht,
K für Wasserstoff, Carrier-System, löslichkeitsvermittelnde Gruppe oder Acylrest einer natürlichen oder unnatürlichen Aminosäure (C-verknüpfte Aminosäure) steht,
T für Hydroxy, Carrier-System, löslichkeitsvermittelnde Gruppe oder amidisch (N-) verknüpfte natürliche oder unnatürliche Aminosäure steht,
R und R' jeweils unabhängig voneinander für OH, Methyl oder Benzyl bedeutet,
r für 0 oder 1 steht und
s für einen Wert von 2 bis 15 steht.

4. Arzneimittel enthaltend eine oder mehrere Verbindungen gemaß den Ansprüchen 1 bis 3.
